Europäisches Patentamt

European Patent Office

Office européen des brevets

(19)

(11) Publication number: **0 015 379**
**B1**

(12) # EUROPEAN PATENT SPECIFICATION

(45) Date of publication of patent specification: **18.05.83**

(21) Application number: **80100446.6**

(22) Date of filing: **29.01.80**

(51) Int. Cl.³: **B 01 J 31/40,**
**C 07 C 45/50, C 07 C 47/02**

(54) **Regeneration of rhodium hydroformylation catalysts.**

(30) Priority: **12.02.79 US 11604**

(43) Date of publication of application:
**17.09.80 Bulletin 80/19**

(45) Publication of the grant of the patent:
**18.05.83 Bulletin 83/20**

(84) Designated Contracting States:
**DE FR GB NL**

(56) References cited:
**EP - A - 0 000 685**
**FR - A - 2 258 357**
**US - A - 3 547 964**

(73) Proprietor: **EASTMAN KODAK COMPANY**
**Patent Department 343 State Street**
**Rochester, New York 14650 (US)**

(72) Inventor: **Dawes, John Leslie**
**P.O. Box 7444**
**Longview Texas (US)**
Inventor: **Devon Jr., Thomas James**
**P.O. Box 7444**
**Longview Texas (US)**

(74) Representative: **Brandes, Jürgen, Dipl.-Chem. Dr.**
**et al,**
**Thierschstrasse 8**
**D-8000 München 22 (DE)**

## Regeneration of rhodium hydroformylation catalysts

This invention relates to the Oxo process for the hydroformylation of an unsaturated compound, such as an $\alpha$-olefin, by the reaction of the unsaturated compound with carbon monoxide and hydrogen in the presence of a catalyst. More specifically, this invention relates to a method of regenerating a rhodium catalyst useful in such a hydroformylation reaction.

In a hydroformylation reaction, which is called the Oxo process, an unsaturated compound, such as an $\alpha$-olefin, is reacted with synthesis gas which contains carbon monoxide and hydrogen to form an aldehyde of the unsaturated compound. For example, propylene is reacted with carbon monoxide and hydrogen in the presence of a suitable catalyst to form isobutyraldehyde and n-butyraldehyde.

Cobalt catalysts are known to be efficient and effective catalysts for the hydroformylation reaction. However, the cobalt catalysts produce undesirable quantities of by-products such as paraffins, alcohols, acetals, aldol type products and other high boiling products. In contrast, rhodium catalysts are known to be more selective in the hydroformylation process and to produce yields of the desired aldehydes as high as 99% and substantially less of the undesired by-products. Also, the rhodium catalysts can be used at much lower temperatures and pressures than the cobalt catalysts and as a result significantly lower equipment and utilities requirements are needed to build and operate a hydroformylation facility using rhodium catalysts.

The rhodium catalysts that are used in a hydroformylation reaction are complexes of rhodium in combination with a ligand or functional group that activates rhodium into a hydroformylation catalyst under reaction conditions. There are many known ligands that can be used in the rhodium catalyst complex. For example, triaryl, trialkyl or mixed aryl alkyl amines, phosphines, arsines or stilbines can be used. The complex of rhodium and ligand reacts with the carbon monoxide and hydrogen in the synthesis gas to become even more complicated. For example, when rhodium and trimethylphosphine are reacted with carbon monoxide and hydrogen, a complex material whose structure is unknown is formed.

During hydroformylation reactions, rhodium catalysts become deactivated to a point where they have insufficient activity to support an economical reaction. Normally a fully active rhodium catalyst complex is straw colored while an inactive complex is black. Monitoring this color change is one practical method of determining if a catalyst is active or inactive.

The principal disadvantages of rhodium catalysts for a hydroformylation reaction are the extremely high capital investment needed for the initial catalyst charge and the lack of an effective method for catalyst reactivation without significant loss of the valuable rhodium. Several methods of regeneration have been suggested in the prior art. U.S. Patent 3,555,098 described the use of water or caustic as a wash for the catalyst. This method removes such poisons as acids but has not been effective for regeneration of the rhodium catalyst. Japanese Patent 73/43799 describes reduction with hydrogen as a suitable regeneration method. Japanese Patent 74/94385 describes the use of molecular oxygen for regeneration of the catalyst.

European patent application 0 00685 describes the treatment of a rhodium triaryl phosphite complex catalyst, which comprises an oxidation reaction at a temperature of from 0 to 85°C until the ligand is completely oxidized.

French patent 22 58 357 describes a regeneration method according to which an organic solution containing the complex of rhodium in combination with triphenylphosphine is submitted to a treatment with an aldehyde such as formaldehyde or isobutyraldehyde in presence of an acid.

None of these methods restores fresh catalyst activity without significant loss of rhodium.

Numerous catalyst regeneration procedures have been tried. These procedures included treating the inactive catalyst with hydrogen, carbon monoxide, synthesis gas (the combination of carbon monoxide and hydrogen used in the Oxo reaction); washing the inactive catalyst with water or aqueous caustic; refluxing the inactive catalyst with aqueous caustic or an acetic acid anhydride mixture; treating the inactive catalyst with hydrochloric acid followed by a sodium isobutyrate wash; treating the inactive catalyst with reducing agents, e.g., sodium borohydride in methanol or hydrazine in ethanol; and treating the inactive catalyst with air in the absence of aldehyde. None of these procedures proved adequate as a catalyst reactivation method.

The process of this invention for regenerating a deactivated rhodium hydroformylation catalyst which has been removed from the hydroformylation reaction, overcomes the problems of the prior art. The method of this invention is characterized by

a) adding aldehyde to the deactivated catalyst so that at least one mole of aldehyde is present for each mole of rhodium and each mole of ligand,

b) oxidizing the aldehyde-containing catalyst with an oxygen-containing gas at a temperature below the boiling point of the aldehyde until substantially all the ligand is oxidized,

c) removing solid oxidized ligand formed during the oxidation, and

d) adjusting the ligand to rhodium ratio of the regenerated catalyst for use in a hydroformylation reaction.

Optionally, it may be desirable to remove some ligand from the catalyst prior to the adjustment of the aldehyde content. This will serve to reduce the amount of ligand lost during oxidation and to reduce the amount of aldehyde which must be added to achieve the correct aldehyde/rhodium/ligand balance. In addition, following oxidation, it may be desirable to treat the catalyst so as to remove any acid produced during the oxidation step. This method results in a virtually complete reactivation of catalyst with minimal rhodium loss and it can be used repeatedly on the same catalyst charge without the build-up of undesirable by-products. Surprisingly it has been found that this method is also effective in reactivating rhodium oxo catalysts which have been poisoned by sulfur.

The reactivation of the deactivated rhodium catalyst is carried out under mild reaction conditions. Consequently, the reactivation of the catalyst can be accomplished in inexpensive equipment using ambient or atmospheric conditions. An oxygen-containing gas, such as air, is used for the oxidation and the oxidation reaction is carried out at a temperature below the boiling point of the aldehyde that is added to the deactivated catalyst. Preferably, the oxidation reaction is carried out at room temperature.

The aldehyde that is added to the deactivated catalyst is preferably an aldehyde that is formed in the hydroformylation reaction. When an $\alpha$-olefin is being reacted in the hydroformylation process, a saturated aliphatic aldehyde such as acetaldehyde, propionaldehyde, n-butyraldehyde, isobutyraldehyde or higher boiling aldehydes can be used. When propylene is being hydroformylated to form butyraldehydes, either the iso or normal butyraldehyde can be used in the catalyst reactivation. The amount of aldehyde that is used is at least equivalent on a mole basis to the amount of rhodium and ligand that is present in the catalyst. Thus, a mole of rhodium and a mole of ligand would require about 2.05 moles of aldehyde. When a catalyst is formed from 1 mole of rhodium and as much as 19 moles of ligand, such a triphenylphosphine, it is preferred to remove some of the triphenylphosphine from the deactivated catalyst before aldehyde is added for the oxidation reaction. Thus, less aldehyde is required for the oxidation reaction and less triphenylphosphine is converted to the corresponding oxide during the oxidation reaction.

In a more specific illustration, a rhodium catalyst utilized in a process such as that described in U.S. Patent 3,527,809 can be removed as a slip or side stream from the process. For each mole of rhodium and ligand present in the side stream an equal molar amount of aldehyde plus a slight excess of aldehyde is added. The aldehydes are preferably iso- or normal butyraldehydes or mixtures thereof. Air is then blown through the solution at a slow rate so the temperature of the mixture does not rise rapidly. As catalyst reactivation proceeds the solution changes from a black color to a straw color. From a few minutes (30 or less) to 48 hours or more are needed for the reactivation. Excess aldehyde and any acids formed during oxidation are then removed. The amount of aldehyde and air used in the oxidation are controlled to minimize the formation of acid in the regeneration process. The solution is then filtered to remove any solids, for example triphenylphosphine oxide, and returned to the catalyst recycle stream with added phosphine. Progress of the catalyst regeneration can be monitored by the change in color from the dark deactivated form to the straw yellow of the active form. Regeneration is not complete until all the phosphine, etc., is oxidized to the phosphine oxide. When excess phosphine is employed in the reaction it should be removed from the stream prior to regeneration and then replaced after the regeneration.

In the following example triphenylphosphine is represented by the symbol $P\phi_3$.

Example

A sample of an oxo catalyst containing 1 mole of rhodium and 19 moles of triphenyl phosphine, deactivated in propylene hydroformylation at 100°C. and 6792 kPa, was removed from the reactor, stripped in vacuo and divided into four portions. The first portion was diluted with isobutyraldehyde to 55 ppm (parts per million) rhodium and tested for propylene hydroformylation at 100°C. and 6792 kPa. Butyraldehydes were produced at a rate of 11 pounds per cubic foot (176 g/liter) per hour and the deactivated catalyst had only 22 percent of the activity of the fresh catalyst.

The second portion of catalyst was diluted with isobutyraldehyde to 55 ppm rhodium, 19 g moles of $P\phi_3$ were added per g-atom of rhodium and tested for propylene hydroformylation at 100°C. and 6792 kPa. Butyraldehyde was produced at the same rate as the previous run showing that addition of $P\phi_3$ did not enhance the activity of the deactivated catalyst.

The two remaining portions of deactivated catalyst were combined, diluted with isobutyraldehyde and treated at room temperature with a slow stream of air for 48 hours. The solution changed from black to a straw yellow and all the triphenylphosphine was oxidized to triphenylphosphine oxide. The solvent was stripped in vacuo at 100°C. to remove the isobutyric acid produced in the oxidation. The residue was diluted with isobutyraldehyde to 55 ppm rhodium and divided into two parts. The first part was tested for propylene hydroformylation at 100°C. and 6792 kPa and produced butyraldehydes at a rate of 224 g/liter per hour showing that oxidized catalyst without added $P\phi_3$ was only 27 percent as active as fresh catalyst.

The second part of the combined catalyst was treated with 19 equivalents of triphenylphosphine per equivalent of rhodium and tested in a similar manner. This catalyst produced butyraldehydes at 51 pounds per cubic foot (816,5 g/liter) per hour which was equivalent to the fresh catalyst under identical conditions.

## Claims

1. A method for regenerating a rhodium-ligand catalyst, that has been deactivated in a hydroformylation reaction, characterized by
    a) adding aldehyde to the deactivated catalyst so that at least one mole of aldehyde is present for each mole of rhodium and each mole of ligand,
    b) oxidizing the aldehyde-containing catalyst with an oxygen-containing gas at a temperature below the boiling point of the aldehyde until substantially all the ligand is oxidized,
    c) removing solid oxidized ligand formed during the oxidation, and
    d) adjusting the ligand to rhodium ratio of the regenerated catalyst for use in a hydroformylation reaction.

2. The method according to Claim 1 wherein the ligand is triphenylphosphine.

3. The method according to Claim 1 wherein the aldehyde is isobutyraldehyde.

4. The method according to Claim 1 wherein the aldehyde-containing catalyst is oxidized with air at room temperature.

## Revendications

1. Procédé pour la régénération d'un catalyseur, contenant un complexe d'un coordinat et de rhodium, qui a été désactivé dans une réaction d'hydroformylation, caractérisé en ce que (a) on ajoute un aldéhyde au catalyseur désactivé de manière qu'au moins une mole d'aldéhyde soit présente pour chaque mole de rhodium et chaque mole de coordinat, (b) on oxyde le catalyseur qui contient de l'aldéhyde avec un gaz contenant de l'oxygène à une température inférieure à la température d'ébullition de l'aldéhyde jusqu'à ce que pratiquement tout le coordinat soit oxydé (c) on élimine les produits solides, résultant de l'oxydation du coordinat, formés pendant l'oxydation et (d) on ajuste le rapport du coordinat au rhodium du catalyseur régénéré pour son utilisation dans une réaction d'hydroformylation.

2. Procédé conforme à la revendication 1, dans lequel le coordinat est la triphènylphosphine.

3. Procédé conforme à la revendication 1, dans lequel l'aldéhyde est l'isobutyraldéhyde.

4. Procédé conforme à la revendication 1, dans lequel le catalyseur qui contient de l'aldéhyde est oxydé par l'air à la température ambiante.

## Patentansprüche

1. Ein Verfahren zum Regenerieren eines Liganden aufweisenden Rhodium-Katalysators, der durch Verwendung in einer Hydroformylierungsreaktion deaktiviert wurde, gekennzeichnet durch:
    a) Zusatz von Aldehyd zu dem deaktivierten Katalysator, so daß mindestens ein Mol Aldehyd für jedes Mol Rhodium und jedes Mol Liganden vorliegt;
    b) Oxidieren des Aldehyd enthaltenden Katalysators mit einem Sauerstoff enthaltenden Gas bei einer Temperatur unterhalb der Siedetemperatur des Aldehydes bis praktisch sämtlicher Ligand oxidiert ist;
    c) Entfernung des während der Oxidation gebildeten festen oxidierten Liganden und
    d) Einstellung des Verhältnisses von Liganden zu Rhodium des regenerierten Katalysators für die Verwendung in einer Hydroformylierungsreaktion.

2. Verfahren nach Anspruch 1, dadurch gekennzeichnet, daß der Ligand Triphenylphosphin ist.

3. Verfahren nach Anspruch 1, dadurch gekennzeichnet, daß der Aldehyd Isobutyraldehyd ist.

4. Verfahren nach Anspruch 1, dadurch gekennzeichnet, daß der Aldehyd enthaltende Katalysator bei Raumtemperatur mit Luft oxidiert wird.